# EUROPÄISCHE PATENTSCHRIFT

(11) **EP 2 997 987 B1**
(45) Veröffentlichungstag und Bekanntmachung des Hinweises auf die Patenterteilung: **08.11.2017**
(21) Anmeldenummer: 15185403.1
(22) Anmeldetag: 16.09.2015
(51) Int. Cl.: A61M 1/14

(54) **DIALYSEMASCHINE**
DIALYSIS MACHINE
MACHINE DE DIALYSE

(30) Priorität: 17.09.2014 DE 102014113368
(43) Veröffentlichungstag der Anmeldung: 23.03.2016
(73) Patentinhaber: B. Braun Avitum AG, 34212 Melsungen (DE)
(72) Erfinder: Ritter, Kai-Uwe, 91126 Rednitzhembach (DE); Stenzel, Bruno, 34346 Hann. Münden (DE)
(74) Vertreter: Winter, Brandl, Fürniss, Hübner, Röss, Kaiser, Polte - Partnerschaft mbB

(56) Entgegenhaltungen:
- WO-A1-2008/117899
- DE-A1-102011 010 249
- KR-A- 20090 078 598
- US-A1- 2004 064 080
- US-A1- 2009 012 455
- US-A1- 2009 284 108

## Beschreibung

Die vorliegende Erfindung betrifft eine Dialysemaschine und insbesondere das Gehäuse einer solchen Dialysemaschine mit einer integrierten Funktions-/ Steuerungsanzeigeeinheit, vorzugsweise in Form eines Monitors oder Touchscreens.

### Technischer Hintergrund

Dialysemaschinen repräsentieren medizinische Geräte, mit denen Blut eines Patienten extrakorporal behandelt werden kann, wenn z.B. dessen Nierenfunktion eingeschränkt ist. Dazu ist an einer solchen Dialysemaschine ein Dialysator angeordnet, der einerseits von dem zu reinigenden Blut des Patienten und andererseits von einer Dialysierflüssigkeit durchströmt wird, wobei gewisse gelöste Substanzen (z.B. Harnstoff) aus dem Blut an die Dialysierflüssigkeit übergehen. Über Schlauchleitungen wird hierfür das zu reinigende Blut vom Patienten zum Dialysator und das gereinigte Blut zurück zum Patienten geleitet. Über andere Leitungen wird die frische Dialysierflüssigkeit ebenfalls zum Dialysator geleitet und verbrauchte Dialysierflüssigkeit aus dem Dialysator entsorgt.

Dialysemaschinen dieser Gattung existieren in Form von stationären (ortsfesten) aber auch mobilen Stationen, wobei insbesondere bei der letztgenannten Variante das Maschinengehäuse mittels daran montierter Rollen verschoben werden kann. Das Gehäuse einer Dialysemaschine hat an seiner Vorderseite meistens oberhalb des Gehäuses einen Monitor oder auch Touchscreen, an dem zumindest die aktuellen Betriebsdaten der Maschine abgelesen und ggf. Maschinen- und/oder Patientendaten für einen ordnungsgemäßen Betrieb eingegeben werden können.

Dabei ist es aus dem Stand der Technik bekannt, den Monitor in die Vorderseite zu integrieren oder an der Oberseite des Gehäuses adaptiv zu installieren.

Bei einer gehäusefesten Integration des Monitors in die Vorderseite des Gehäuses ist eine Öffnung der Vorderseite zu Servicezwecken in der Regel nicht möglich. Daher werden Serviceklappen an der Rückseite oder an Seitenteilen des Maschinengehäuses vorgesehen. Außerdem ist eine ergonomische Positionierung und Ausrichtung des Monitors schwierig, da neben dem Monitor/Touchscreen weitere Funktionselemente, wie bspw. Pumpen, Sensoren, Schlauchführungen etc. an der Maschinenvorderseite angeordnet sind, welche den Platz zur Anbringung des Monitors/Touchscreens einschränken.

Bei der adaptiven Installation oberhalb des Gehäuses bzw. auf der oberen Gehäusehaube ergeben sich schwer zu reinigende Zwischenräume zwischen dem Monitor/Touchscreen und dem Gehäuse. Wenn der Monitor/Touchscreen zusätzlich auf einen Drehkranz als Montagesockel aufgesetzt ist, muss dessen Schnittstelle zum Gehäuse aufwändig abgedichtet werden und es sind ggf. zusätzliche EMV-Maßnahmen nötig.

### Stand der Technik

Aus dem Stand der Technik ist eine Dialysemaschine dieser Gattung bekannt, deren Gehäuse in einem Abschnitt der Gehäusevorderseite eine Fronttür hat, und deren Monitor/Touchscreen oberhalb dieser Fronttür in einem anderen Abschnitt der Gehäusevorderseite unterhalb der oberen Gehäuseabdeckhaube/Deckel befestigt ist. Dabei ist das Gehäuse des Monitors/Touchscreens integriert mit dem Gehäuse der Dialysemaschine ausgebildet oder ist adaptiv an die Gehäusevorderseite angeschraubt. Der Monitor/Touchscreen verbleibt daher gehäusefest oberhalb der Fronttür, auch wenn diese geöffnet wird und kann somit grundsätzlich nur von vorn bezüglich der Maschine abgelesen werden.

Nachteilig an derartigen Dialysemaschinen ist, dass die Vorderseite der Dialysemaschine durch eine Trennfuge unterbrochen ist. D. h. die Vorderseite des Maschinengehäuses ist in zwei Abschnitte unterteilt, nämlich in einen unteren Abschnitt, ausgebildet durch die Gehäusetür und einen oberen Abschnitt, ausgebildet durch eine fixe Gehäusewand als Träger/Montagefläche für Bedienelemente, Anzeigen und den Monitor/Touchscreen der Dialysemaschine. Zwischen den beiden Abschnitten bildet sich zwangsläufig eine nach vorn sichtbare Trennfuge aus, in der sich Schmutz festsetzen kann.

Aus den Druckschriften WO 2008 / 117899 A1 und KR 2009 0078598 A ist jeweils eine Blutbehandlungsmaschine mit einer regulären Tür offenbart, in welcher ein Monitor integriert ist. Gemäß der Druckschrift WO 2008 / 117899 A1 ist die Tür hierbei als Schiebetür ausgebildet, die bei einem Öffnen der Tür über den Corpus (d.h. den Hauptabschnitt) der Blutbehandlungsmaschine hinaus verschoben werden kann. Gemäß der Druckschrift KR 2009 0078598 A ist es vorgesehen, dass die Tür mit dem integrierten Monitor mittels mehrerer Scharniere zum Öffnen aufgeklappt werden kann.

Gemäß der Druckschrift US 2009 / 0284108 A1 ist der Monitor über einen dreh-/ verschwenkbaren Montagesockel auf der Haube des Gehäuses der Blutbehandlungsmaschine montiert und daher nicht in einer Fronttür der Dialysemaschine integriert.

Weiterhin offenbart die Druckschrift US 2009 / 0012455A1 eine Dialysemaschine mit einem aufklappbaren Monitor. Diese Dialysemaschine hat jedoch keine Fronttür.

Aus der Druckschrift DE 102011010249 A1 ist weiterhin eine Dialysemaschine mit einem besonderen Griff bekannt.

Zudem offenbart die Druckschrift US 2004 / 0064080 A1 eine Dialysemaschine mit einem direkt an dem Korpus der Dialysemaschine montierten Monitor. Bei dieser Dialysemaschine sind die Türen seitlich angeordnet.

Aus dem Stand der Technik ist aber auch eine andere Dialysemaschine dieser Gattung bekannt, deren Maschinengehäuse an der Vorderseite eine Gehäusetür aufweist, welche sich über die gesamte Gehäuseseite zwischen Gehäuseboden und Gehäusehaube erstreckt. In diesem Fall ist der Monitor/Touchscreen über einen vorzugsweise drehbaren Montagesockel oder Fuß an/auf der Gehäusehaube verschraubt. Der Montagesockel erlaubt zwar ein Schwenken und Drehen des Monitors/Touchscreen derart, dass dieser aus allen Richtungen/von allen Seiten des Maschinengehäuses gut ablesbar ist. Indessen nimmt aber der Montagesockel einen großen Flächenanteil der Maschinenhaube in Anspruch, sodass diese als Ablagefläche für diverse Gegenstände nicht mehr zur Verfügung steht.

### Kurzbeschreibung der Erfindung

Dem gegenüber liegt der Erfindung die Aufgabe zu Grunde, eine Dialysemaschine mit Monitor/Touchscreen und Fronttür zu schaffen, deren Vorderseite flächig ohne schwer zu reinigende vordere Trennfuge gestaltet und gefertigt werden kann. Ein bevorzugtes Ziel ist es, die Ablesbarkeit/Bedienbarkeit des Monitors/Touchscreens von unterschiedlichen Seiten der Maschine her zu ermöglichen. Schließlich ist es ein weiteres bevorzugtes Ziel, die von der oberen Gehäusehaube gebotene Fläche als Ablagefläche für unterschiedliche Gegenstände insbesondere die Dialysebehandlung betreffend zu erhalten.

Diese Aufgabe sowie die weiteren Ziele werden gelöst bzw. erreicht durch eine Dialysemaschine mit den Merkmalen des Patentanspruchs 1. Weitere vorteilhafte Ausgestaltungen der Erfindung sind in den abhängigen Patentansprüchen beschrieben.

Der Grundgedanke der Erfindung besteht im Wesentlichen darin, die Fronttür des Maschinengehäuses zumindest bereichsweise über die obere Gehäusehaube/Gehäusedeckel hinaus zu verlängern. Der dadurch entstehende Türverlängerungsabschnitt, der über das Maschinengehäuse/Gehäusehaube schild-/tafelartig hinausragt und daher eigentlich keine Türfunktion zum Verschließen einer Gehäuseöffnung hat, dient als Montage-/Einbauabschnitt für den Monitor/Touchscreen. Der Monitor/Touchscreen hat somit die ergonomisch richtige Position/Höhe für gute Ables-/Bedienbarkeit und kann zudem zusammen mit der Fronttür verschwenkt werden, um so auch aus einer seitlichen oder rückwärtigen Richtung bezüglich des Maschinengehäuses abgelesen/bedient werden zu können. Da darüber hinaus der Monitor/Touchscreen an der Fronttür montiert bzw. in diese integriert ist, bleibt die gesamte Gehäusehaube als Ablagefläche erhalten.

Die beanspruchte Dialysemaschine weist im Konkreten ein Gehäuse auf, das einen z.B. schrankartigen Hauptabschnitt hat, an dessen Vorderseite eine Fronttür angeordnet ist. Dabei kann die Fronttür die gesamte Vorderseite des schrankartigen Gehäuse-Hauptabschnitts vorzugsweise mit Ausnahme eines ggf. zurückversetzten Gehäusesockels abdecken/bilden. Weiterhin hat die Dialysemaschine einen Monitor/Touchscreen, der zumindest abschnittsweise oberhalb des Hauptabschnitts des Gehäuses angeordnet ist. Erfindungsgemäß ist der Monitor/Touchscreen an der Fronttür befestigt oder in diese integriert. Damit kann die gesamte Vorderseite ggf. mit Ausnahme des Gehäusesockels flächig und einheitlich ohne Trennfuge gestaltet und einfach gefertigt werden. Es entfällt eine schwer zu reinigende Stelle/Nut an der Vorderseite der Dialysemaschine.

Insbesondere wenn die Fronttür an einer Seite des Gehäuse-Hauptabschnitts mittels geeigneter Scharniere oder Scharnierbänder angeschlagen ist, kann diese über mindestens 90° vorzugsweise etwa 180° aufgeschwenkt werden. Damit kann der Monitor/Touchscreen auch von einer Person abgelesen werden, die sich an der Rückseite der Dialysemaschine befindet und z.B. Wartungsarbeiten durchführt.

Bei einer vorrichtungstechnisch einfachen Weiterbildung ist ein Monitorgehäuse oder Monitoraufnahmefach einstückig mit der Fronttür gebildet. In diesem Fall ist die Rückseite des Monitors/Touchscreens von der Fronttür schützend verdeckt, wohingegen der Monitor/Touchscreen bündig oder mit nur geringem Überstand an der Vorderfläche der Fronttür abschließt.

Wenn die Fronttür aus Blech gefertigt ist, kann diese an ihrem Rand einfach zu Stabilisierungsstegen oder Bändern umgebogen/gefaltet sein. Damit kann die Fronttür das Gehäuse/dessen Hauptabschnitt im geschlossenen Zustand außenseitig umgreifen, sodass die Trennfuge zwischen Tür und Gehäuse von vorn nicht sichtbar ist. Damit wird das optische Erscheinungsbild der Dialysemaschine verbessert.

Um Schmutzecken zu vermeiden wird es bevorzugt, wenn das Monitorgehäuse und die Fronttür etwa eine gleiche Breite haben.

Vorzugsweise erstreckt sich das gesamte Monitorgehäuse oder das MonitorAufnahmefach oberhalb der Oberseite des Hauptabschnitts des Gehäuses, d.h. oberhalb der oberen Gehäusehaube. Wird daher die Fronttür geschlossen, derart, dass deren Vorderplatte am Gehäuse anliegt, übergreift der Monitor/das Monitoraufnahmefach entsprechend der Monitor-Gehäusetiefe die Gehäusehaube geringfügig. Dadurch entsteht zwischen der Fronttür und dem Gehäuse eine Trennfuge, die einheitlich auf einer Ebene um das gesamte Gehäuse führt, ohne das Ecken ausgebildet werden.

Bei einer besonders bevorzugten Weiterbildung der erfindungsgemäßen Dialysemaschine ist an einer Oberseite des Hauptabschnitts des Gehäuses (Gehäusehaube) eine im Wesentlichen ebene Ablagefläche z.B. für Behälter mit Dialysezubehör gebildet oder angeordnet. Diese ist hinter dem Monitor/Touchscreen und damit tiefer als bei Ablagen gemäß dem Stand der Technik angeordnet. Somit wird die Ergonomie verbessert. Wenn die Fronttür geöffnet und somit der Monitor/Touchscreen weggeschwenkt ist, ist die Ablagefläche besonders einfach auch von der Vorderseite zugänglich.

Bei einer ersten erfindungsgemäßen Variante erstreckt sich die Ablagefläche im Wesentlichen über die gesamte Fläche der Gehäuseoberseite/Gehäusehaube. Bei einer zweiten erfindungsgemäßen Variante hat die Ablagefläche eine Umrandung, durch die ein Herunterfallen z.B. der Behälter mit Dialysezubehör verhindert wird.

Aus optischen Gründen kann eine obere Rahmenfläche der Umrandung etwa senkrecht zum Monitor oder zur Fronttür ausgerichtet sein. Wenn also z.B. der Monitor oder die Fronttür senkrecht ausgerichtet ist, ist die obere Rahmenfläche der Umrandung waagerecht ausgerichtet. Aus ergonomischen Gründen wird es bevorzugt, wenn der Monitor und/oder die Fronttür eine von der Senkrechten abweichende Neigung haben, sodass daran montierte Betätigungs- und Ableseeinrichtungen besser zugänglich sind. Aus optischen Gründen sollten die Neigungen des Monitors und der Fronttür etwa gleich sein. Bei der oben genannten zweiten Variante der Ablage wird es aus optischen Gründen bevorzugt, wenn dann die obere Rahmenfläche der Umrandung die gleiche Neigung zur Waagerechten hat, wie der Monitor und/oder die Fronttür zur Senkrechten. Damit ist darüber hinaus sicher gestellt, dass beispielsweise Tropfwasser nach hinten weg vom Monitor und den Bedienelementen läuft und sich nicht in der Trennfuge zwischen Fronttür und Gehäusehaube sammelt. Eventuelle Lackbeschädigungen z.B. durch Behälter mit Dialysezubehör, die auf der Ablage abgestellt werden, werden durch die geneigte obere Rahmenfläche zusätzlich verdeckt.

Bei einer bevorzugten Ausgestaltung der zweiten Variante erstreckt sich die Umrandung im Bereich des Monitors bzw. Monitorgehäuses und an den beiden Seiten der Oberseite. Damit ist die Umrandung U-förmig und an einer Rückseite des Hauptabschnitts des Gehäuses offen.

Seitliche Fugen zwischen dem Hauptabschnitt des Gehäuses und seiner Fronttür haben infolge der abgebogenen Stabilisierungsleisten/Bänder vorzugsweise maximal 6 cm - insbesondere etwa 4 cm - Abstand zur Vorderseite des Hauptabschnitts.

Im Folgenden wird anhand der anliegenden Figuren ein bevorzugtes Ausführungsbeispiel der Erfindung detailliert beschrieben. Es zeigen
Figur 1 ein bevorzugtes Ausführungsbeispiel der erfindungsgemäßen Dialysemaschine in einer perspektivischen Frontansicht;
Figur 2 das bevorzugte Ausführungsbeispiel der erfindungsgemäßen Dialysemaschine in einer Seitenansicht;
Figur 3 das bevorzugte Ausführungsbeispiel der erfindungsgemäßen Dialysemaschine in einer perspektivischen Rückansicht; und
Figur 4 einen Ausschnitt aus Figur 3.

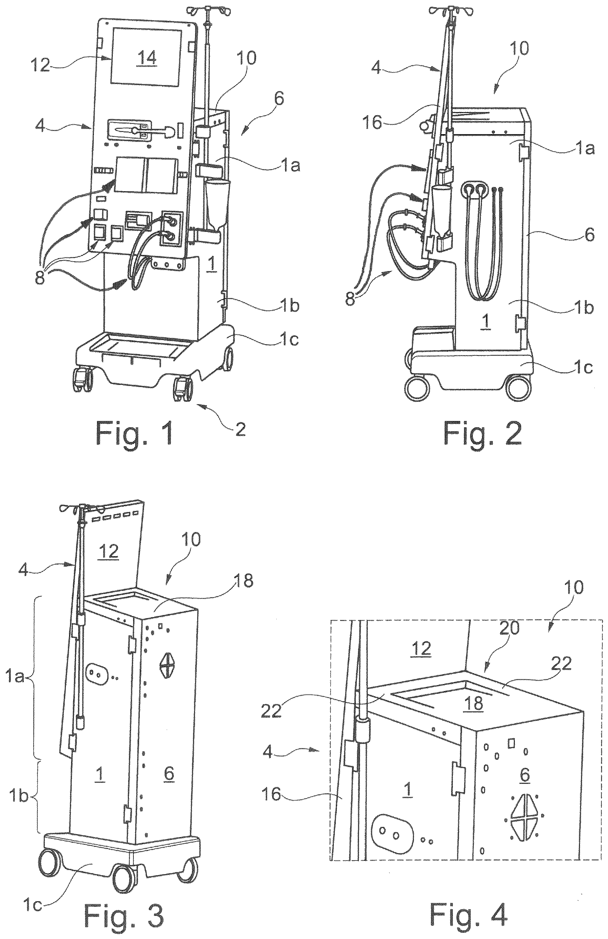

Figur 1 zeigt eine perspektivische Frontansicht der erfindungsgemäßen Dialysemaschine. Sie hat ein Gehäuse 1 mit einem schrankartigen, etwa quaderförmigen Hauptabschnitt 1a, der auf einem bezüglich der Vorderseite des Hauptabschnitts 1a zurückgesetzten Gehäusesockel 1b ruht, der wiederum auf einer Plattform 1c aufgesetzt ist. An der Unterseite der Plattform 1c sind drei oder vier Rollen 2 angebracht sind, über die die Dialysemaschine verschoben werden kann.

In einer Vorderseite des Hauptabschnitts 1a ist eine Fronttür 4 vorgesehen, die vorliegend die Vorderseite des Hauptabschnitts 1a ganzflächig überdeckt, während an einer Hinterseite des Gehäuses 1 eine rückwärtige Tür 6 vorgesehen ist. Die Fronttür 4 ist um einige Grad schräg zur Senkrechten angestellt, so dass an ihr angeordnete Funktionselemente 8, wie bspw. Pumpen, Sensoren, Schlauchführungen etc. entsprechend leicht nach oben gerichtet sind.

Die Fronttür 4 ragt im vorliegenden Ausführungsbeispiel vorzugsweise um etwa ein Drittel ihrer Höhe über eine Oberseite/Gehäusehaube 10 des Hauptabschnitts 1a des Gehäuses 1 hinaus. In diesem Abschnitt bildet die Fronttür 4 ein Monitor-/ Touchscreen-Gehäuse bzw. Aufnahmefach 12 aus, in dem ein Monitor oder Touchscreen 14 (nachfolgend nur noch als Monitor bezeichnet) aufgenommen ist. Der Monitor 14 ist gemeinsam mit der Fronttür 4 und den darauf angeordneten Funktionselementen 8 um einige Grad zur Senkrechten angestellt, und ist so der Sichthöhe einer Bedienperson durchschnittlicher Größe optimal entgegen gerichtet.

Die Fronttür 4 ist gemäß der Fig. 1 an einer Seitenfläche des Gehäusehauptabschnitts 1a schwenkbar angeschlagen und kann daher um mindestens 180° aus ihrer Schließposition heraus aufgeschwenkt werden, wie dies nachfolgend noch näher beschrieben wird.

Figur 2 zeigt die Dialysemaschine gemäß Figur 1 in einer Seitenansicht. Die Fronttür 4 mit dem Monitorgehäuse 12 ist einstückig aus Blech gefertigt und hat einen etwa 4 cm breiten Rand bzw. Abkantung 16, der durch Umbiegen des Blechs um 90° nach hinten hergestellt ist. Da das Monitorgehäuse/Aufnahmefach 12 eine Tiefe von etwa 4 cm aufweist, und da eine Breite des Monitorgehäuses/Aufnahmefachs 12 derjenigen der Fronttür 4 entspricht, ist das Monitorgehäuse 12 formtechnisch und fertigungstechnisch optimal in die Fronttür 4 integriert. Damit ist die gesamte an der Fronttür 4 gebildete Bedienfront optimal flächig, einheitlich und quasi fugenfrei.

Figur 3 zeigt die Dialysemaschine der vorhergehenden Figuren in einer perspektivischen Rückansicht. Wenn beispielsweise eine Person über die rückwärtige Tür 6 an der Dialysemaschine beschäftigt ist, kann sie die Fronttür 4 um etwa 180° gemäß vorstehender Beschreibung aufschwenken und den Monitor 14 (vgl. Fig. 1) aus rückwärtiger Richtung ablesen. Da die Fronttür 4 geneigt am Hauptabschnitt 1a angeschlagen ist, ist der Monitor 14 im aufgeschwenkten Zustand der Fronttür 4 leicht nach unten geneigt und kann so von einer zu Wartungszwecken gebeugten oder knienden Person gut abgelesen werden.

An der Oberseite/Gehäusehaube 10 des Hauptabschnitts 1a des Gehäuses 1 ist eine im Wesentlichen ebene Ablagefläche 18 ausgebildet, die etwa auf der Höhe der Unterkante des Monitors 14 und damit optimal tief angeordnet ist.

Figur 4 zeigt die Oberseite 10 des Hauptabschnitts 1a des Gehäuses 1. Die dort ausgebildete Ablagefläche 18 ist mit einer dreiseitigen Umrandung 20 versehen. Diese hat eine gewisse Breite, so dass an der Oberseite der Umrandung 20 eine bandförmige Rahmenfläche 22 gebildet ist. Diese ist aus optischen Gründen um den gleichen Winkel zur Waagerechten angestellt, wie die Fronttür 4 zur Senkrechten angestellt ist. Technisch ist die Schrägstellung dazu vorgesehen, Wasser, vorzugsweise Tropfwasser von der Vorderseite des Gehäuses 1 fern zu halten.

Auf der Ablagefläche 18 kann eine rutschhemmende Matte vorgesehen sein, um ein Herabfallen von Gegenständen über die hintere offene Seite zu verhindern.

Offenbart ist eine Dialysemaschine, die ein Gehäuse und einen Monitor aufweist. An einer Vorderseite des Gehäuses ist eine Fronttür angeordnet, an deren Oberseite ein Monitorgehäuse ausgebildet und ein Monitor befestigt ist. Der Monitor ist zumindest abschnittsweise oberhalb einer Oberseite des Gehäuses angeordnet. Hinter dem Monitor an der Oberseite des Gehäuses kann eine vergleichsweise niedrige und damit ergonomische Ablagefläche ausgebildet oder angeordnet sein.

### Bezugszeichenliste

- 1: Gehäuse
- 1a: Hauptabschnitt
- 1b: Sockel
- 1c: Plattform
- 2: Rolle
- 4: Fronttür
- 6: rückwärtige Tür
- 8: Funktionselemente
- 10: Oberseite/Gehäusehaube
- 12: Monitorgehäuse/Aufnahmefach
- 14: Monitor/Touchscreen
- 16: Rand
- 18: Ablagefläche
- 20: Umrandung
- 22: Rahmenfläche

## Patentansprüche

1. Dialysemaschine mit einem Gehäuse (1), das einen Hauptabschnitt (1a) hat, dessen Oberseite eine Gehäusehaube (10) bildet und an dessen Vorderseite eine Fronttür (4) angeordnet ist, und mit einem Monitor oder Touchscreen (14), der zumindest abschnittsweise oberhalb des Hauptabschnitts (1a) positioniert ist, ***dadurch gekennzeichnet, dass*** die Fronttür (4) einen Türverlängerungsabschnitt aufweist, der im geschlossenen Zustand der Fronttür (4) über eine Gehäuseöffnung und auch über die die Gehäusehaube (10) bildende Oberseite des Hauptabschnitts (1a) des Gehäuses (1) nach oben hinausragt, und der Monitor oder Touchscreen (14) an der Fronttür (4) in dem oben hinausragenden Türverlängerungsabschnitt in vorzugsweise integrierter Weise so angeordnet ist, dass sich der Monitor oder Touchscreen auch im geschlossenen Zustand der Fronttür (4) zumindest abschnittsweise oberhalb der Gehäusehaube (10) erstreckt.

2. Dialysemaschine nach Anspruch 1, ***dadurch gekennzeichnet, dass*** die Fronttür (4) zumindest über näherungsweise 90° und vorzugsweise zumindest über näherungsweise 180° aufschwenkbar ist.

3. Dialysemaschine nach einem der vorhergehenden Ansprüche, ***gekennzeichnet durch*** ein Monitorgehäuse oder Aufnahmefach (12) für einen Monitor/Touchscreen, das einstückig mit der Fronttür (4) zumindest abschnittsweise oberhalb der Hauptabschnitts (1a) des Gehäuses (1) ausgebildet ist.

4. Dialysemaschine nach einem der vorhergehenden Ansprüche, ***dadurch gekennzeichnet, dass*** die Fronttür (4) aus Blech gefertigt ist, das am Türumfang zu einer Borde abgekantet ist, deren Breite in etwa der Tiefe des Monitors/Touchscreens (12) oder dessen Aufnahmefachs (12) entspricht.

5. Dialysemaschine nach Anspruch 3 oder 4, ***dadurch gekennzeichnet, dass*** das Monitorgehäuse oder das Aufnahmefach (12) und die Fronttür (4) jeweils eine Breite haben, derart dass die Fronttür einen leistenförmigen Rand oder Rahmen um das Monitorgehäuse oder Aufnahmefach (12) bildet.

6. Dialysemaschine nach einem der vorhergehenden Ansprüche, ***dadurch gekennzeichnet, dass*** an einer Oberseite oder Gehäusehaube (10) des Hauptabschnitts (1a) eine im Wesentlichen ebene Ablagefläche (18) gebildet oder angeordnet ist, deren als Ablage nutzbares Flächenmaß im Wesentlichen der Abmessung des Hauptabschnitts (1a) entspricht.

7. Dialysemaschine nach Anspruch 6 ***dadurch gekennzeichnet, dass*** sich die Ablagefläche (18) über die gesamte Breite der Oberseite (10) erstreckt.

8. Dialysemaschine nach Anspruch 6, ***dadurch gekennzeichnet, dass*** die Ablagefläche (18) eine Umrandung (20) hat.

9. Dialysemaschine nach Anspruch 8, ***dadurch gekennzeichnet, dass*** eine obere Rahmenfläche (22) der Umrandung (20) etwa senkrecht zum Monitor/Touchscreen (14) und/oder zur Fronttür (4) ausgerichtet sind.

10. Dialysemaschine nach einem der vorhergehenden Ansprüche, ***dadurch gekennzeichnet, dass*** der Monitor/Touchscreen (14) und/oder die Fronttür (4) eine von der Senkrechten abweichende Neigung haben.

11. Dialysemaschine nach Anspruch 10, ***dadurch gekennzeichnet, dass** die* Neigungen des Monitors/Touchscreen (14) und der Fronttür (4) etwa gleich sind.

12. Dialysemaschine nach Anspruch 8 und 11, ***dadurch gekennzeichnet, dass*** sich die Umrandung (20) im Bereich des Monitors/Touchscreens (14) und an den beiden Seiten der Oberseite (10) erstreckt.

## Claims

1. Dialysis machine having a housing (1) which includes a main section (1a) whose top side forms a housing cover (10), and on the front side of which a front door (4) is situated, and a monitor or touchscreen (14) which is positioned, at least in portions, above the main section (1a), **characterized in that** the front door (4) comprises an extended door section which, in the closed state of the front door (4), protrudes upwardly beyond a housing opening and also beyond the top side forming the housing cover (10) of the main section (1a) of the housing (1), and the monitor or touchscreen (14) is situated, preferably in an integrated manner, on the front door (4) in the upwardly protruding extended door section, so that the monitor or touchscreen extends, at least in portions, above the housing cover (10) also in the closed state of the front door (4).

2. Dialysis machine according to Claim 1, ***characterized in that*** the front door (4) is pivotable at least over approximately 90°, and preferably at least over approximately 180°.

3. Dialysis machine according to one of the preceding claims, ***characterized by*** a monitor housing or storage compartment (12) for a monitor/touchscreen, which is formed, at least in portions, in one piece with the front door (4) above the main section (1a) of the housing (1).

4. Dialysis machine according to one of the preceding claims, ***characterized in that*** the front door (4) is made of sheet metal, which at the door periphery is folded down to form a rim whose width corresponds approximately to the depth of the monitor/touchscreen (12) or its storage compartment (12).

5. Dialysis machine according to Claim 3 or 4, ***characterized in that*** the monitor housing or the storage compartment (12) and the front door (4) each have a width such that the front door forms a strip-shaped edge or frame around the monitor housing or storage compartment (12).

6. Dialysis machine according to one of the preceding claims, ***characterized in that*** an essentially flat storage surface (18) is formed or situated on a top side or housing cover (10) of the main section (1a), wherein the surface area of the storage surface, which is usable for storage, corresponds essentially to the dimensions of the main section (1a).

7. Dialysis machine according to Claim 6, ***characterized in that*** the storage surface (18) extends over the entire width of the top side (10).

8. Dialysis machine according to Claim 6, ***characterized in that*** the storage surface (18) has a border (20).

9. Dialysis machine according to Claim 8, ***characterized in that*** an upper frame surface (22) of the border (20) is oriented approximately perpendicularly with respect to the monitor/touchscreen (14) and/or the front door (4).

10. Dialysis machine according to one of the preceding claims, ***characterized in that*** the monitor/touchscreen (14) and/or the front door (4) have a non-vertical inclination.

11. Dialysis machine according to Claim 10, ***characterized in that*** the inclinations of the monitor/touchscreen (14) and of the front door (4) are approximately the same.

12. Dialysis machine according to Claims 8 and 11, ***characterized in that*** the border (20) extends in the area of the monitor/touchscreen (14) and at both sides of the top side (10).

## Revendications

1. Machine de dialyse comportant un logement (1) qui a un segment principal (1a), dont le côté supérieur forme un capot du logement (10) et sur le côté avant duquel est disposée une porte frontale (4), et comportant un moniteur ou un écran tactile (14) qui est positionné au moins pour une partie au-dessus du segment principal (1a), **caractérisée en ce que** la porte frontale (4) présente un segment de prolongement de porte qui dépasse vers le haut, à l'état fermé de la porte frontale (4) sur une ouverture du logement et également sur le côté supérieur formant le capot du logement (10), du segment principal (1a) du logement (1) et le moniteur ou l'écran tactile (14) est disposé dans le segment de prolongement de porte dépassant vers le haut de préférence de façon intégrée de telle sorte que le moniteur ou l'écran tactile s'étende également à l'état fermé de la porte frontale (4) au moins pour une partie au-dessus du capot du logement (10).

2. Machine de dialyse selon la revendication 1, **caractérisée en ce que** la porte frontale (4) peut basculer au moins approximativement de 90° et de préférence au moins approximativement de 180°.

3. Machine de dialyse selon l'une des revendications précédentes, **caractérisée par** un logement de moniteur ou un compartiment de réception (12) pour un moniteur/écran tactile, qui est formé d'un seul tenant avec la porte frontale (4) au moins sur une partie, au-dessus du segment principal (1a) du logement (1).

4. Machine de dialyse selon l'une des revendications précédentes, **caractérisée en ce que** la porte frontale (4) est réalisée en tôle qui est repliée sur le pourtour de la porte en une bordure dont la largeur correspond à peu près à la profondeur du moniteur/écran tactile (12) ou à son compartiment de réception (12).

5. Machine de dialyse selon la revendication 3 ou 4, **caractérisée en ce que** le logement du moniteur ou le compartiment de réception (12) et la porte frontale (4) ont respectivement une largeur de telle sorte que la porte frontale forme un bord ou un cadre en forme de baguette autour du logement du moniteur ou du compartiment de réception (12).

6. Machine de dialyse selon l'une des revendications précédentes, **caractérisée en ce que** sur un côté supérieur ou sur le capot du logement (10) du segment principal (1a) est formée ou est disposée une surface de dépôt (18) sensiblement plane dont la superficie utile pour le dépôt correspond sensiblement à la dimension du segment principal (la).

7. Machine de dialyse selon la revendication 6, **caractérisée en ce que** la surface de dépôt (18) s'étend sur la largeur totale du côté supérieur (10).

8. Machine de dialyse selon la revendication 6, **caractérisée en ce que** la surface de dépôt (18) a une bordure périphérique (20).

9. Machine de dialyse selon la revendication 8, **caractérisée en ce qu'**une surface cadre supérieure (22) de la bordure périphérique (20) est orientée à peu près perpendiculairement au moniteur/écran tactile (14) et/ou à la porte frontale (4).

10. Machine de dialyse selon l'une des revendications précédentes, **caractérisée en ce que** le moniteur/écran tactile (14) et/ou la porte frontale (4) ont une inclinaison déviée par rapport à la verticale.

11. Machine de dialyse selon la revendication 10, **caractérisée en ce que** les inclinaisons du moniteur/de l'écran tactile (14) et de la porte frontale (4) sont à peu près identiques.

12. Machine de dialyse selon les revendications 8 et 11, **caractérisée en ce que** la bordure périphérique (20) s'étend dans la zone du moniteur/écran tactile (14) et sur les deux côtés du côté supérieur (10).
